# EUROPEAN PATENT APPLICATION

(11) **EP 4 268 646 A1**
(43) Date of publication of application: **01.11.2023**
(21) Application number: 21915207.1
(22) Date of filing: 23.12.2021
(51) Int. Cl.: A41D 13/002, A41D 13/005

(54) **CLOTHING SYSTEM**

(30) Priority: 28.12.2020 JP 2020218673
(71) Applicant: Nakatsugawa, Shigekazu, Tokyo, 134-0083 (JP)
(72) Inventor: Nakatsugawa, Shigekazu, Tokyo, 134-0083 (JP)
(74) Representative: Dehns
(86) International application number: PCT/JP2021/047967
(87) International publication number: WO 2022/145347

(57) **Abstract**

A garment system includes: a garment that is worn by a user; a blower unit that blows air toward the garment; and a control device that controls the blower unit, wherein the garment is divided into a plurality of sections respectively corresponding to a plurality of parts of a body of the user, and includes an inner layer positioned on a side of the user and an outer layer positioned on a side opposite to the user when viewed from the inner layer, the blower unit blows air between the inner layer and the outer layer of each of the plurality of sections, and the control device controls air blowing performed by the blower unit for each of the sections.

## Description

### Technical Field

The present invention relates to a garment system to be used by a user during sleep.

### Background Art

In recent years, the situations surrounding humankind are changing drastically: extreme climates due to environmental destruction associated with global warming; widening disparities in all aspects due to the global economy; possibility of war due to the intensifying conflict between the U.S. and China; fear of death, especially among the elderly and those with chronic illnesses, due to the worldwide spread of the COVID-19, and so on, all the above situations are rapidly advancing on a global scale. The disparities are not limited to economic power, nutrition, and education, and widely extend to abilities of exercise, information acquisition and processing, and the like. In other words, it is not an exaggeration to say that humankind is in a vortex of unsurpassed violent motion. In such situations, it is quite difficult for people to maintain good sleep that is sufficient in both quantity and quality which are important for maintaining peace of mind and physical health. On the other hand, it has been considered that with aging, sleep, like other vital functions such as reduced physical activity, undergoes changes, so that it is inevitable that sleep time, which is strongly correlated with cognitive function, becomes shorter, shallow sleep increases, and deep sleep decreases. That is, it has been considered that the risk of dementia increases with aging and the shortening of the healthy life span is unavoidable.

The life (daily activity) of people consists of social activity outside the home accounting for about 34 to 50% inclusive in length, home life accounting for about 17 to 33% inclusive in length, and sleep time accounting for the remaining about 33% in length, and is controlled by a biological clock. During sleep, memory is fixed, and sleep plays a major role in maintenance of visceral functions, and it has become clear that sleep is indispensable for improving productivity. In a sense, activities other than sleep time are supported by psychology and physical environments, etc., that is, water intake, nutrition (reflecting digestion, absorption, metabolism, excretion, etc.), exercise, sleep, lifestyle habits, memory, purpose of life, etc., and conversely, sleep reflects physical or psychological fatigue, stress, psychological state, other social activities, nutrition, exercise, lifestyle habits, etc., and its quality and quantity are influenced.

It is also known that sleep varies greatly among individuals in terms of change in body temperature, balance between calorific values (exercise amount, meal content, etc.) and heat emission amounts (perspiration ability, etc.), etc., and is also greatly affected by the climate of the living area, that is, the environment such as temperature, moisture, sound, light, oxygen saturation, carbon dioxide concentration, and carbon monoxide concentration. With regard to individual differences, there are wide and large differences in individual body composition, particularly muscle mass related to calorific value, and skeleton / heat dissipation, cardiovascular system / nervous system related to perspiration, development of internal organs / degree of aging, combination of lifestyle-related diseases, immune / endocrine system balance or the like, and quality / content of work, etc. Actually, the sensible comfortable temperature greatly depends on the moisture even when viewed from the wet bulb globe temperature.

By the way, the temperature of each body part is determined by a balance of
firstly, factors that lead to a temperature rise:
1 import of heat (mainly by arterial blood);
2 thermogenesis (mainly muscle and internal organs (e.g., liver) and hyperemia, inflammatory response);
3 thermal convection (transfer of heat from a hot gas (or liquid) passing over exposed skin to the skin);
4 thermal conduction (transfer of heat from a surface (solid) that is in direct contact and is hotter than body temperature to a colder body surface);
5 suppression of thermal convection and conduction (obesity (reduction in body surface area), thick wear, wind shielding),

   secondly, factors that lead to a temperature drop:
6 heat emission radiation (mainly affected by infrared rays, body surface area (thinness), and autonomic nerve balance to reduce the temperature difference from the surrounding environment);
7 evaporation (e.g., cooling by evaporation of sweat or moisture);
8 thermal convection (transfer of heat to a cold gas (or liquid) passing over exposed skin, for example, ventilation);
9 thermal conduction (conduction of heat from the body surface to a surface (solid) cooler than the body surface temperature, or transfer of heat from a high-temperature surface in direct contact to a low-temperature (e.g., ischemic site) surface);
10 export of heat (mainly by venous blood / cutaneous vein / capillary blood, and ischemia), and so on.

Thermogenesis is due to muscle exercise (60%) and visceral metabolism (40%). In addition, sympathetic excitation, hyperthyroidism, severe obesity, and the like also affect the rise in body temperature. On the other hand, in the cases of parasympathetic nerve dominance, hypothyroidism, and wasting, the body temperature decreases.

It has been reported for a long time that the elderly people or people with severely obesity, frailty, or complications of various diseases, etc., who suffer from serious conditions due to hypothermia or heat stroke during sleep in extremely hot or cold weather, have experienced sudden changes in their health. Proper air conditioning should be able to prevent the sudden changes to a large extent, but in today's abnormal weather, normal air conditioning does not seem to be sufficient. To reconfirm, the mechanism of body temperature retention will be reviewed again.

The thermoregulatory center (hypothalamus of the brain) senses the body temperature and the temperature of organs such as muscles and livers via nerves serving as sensors for the body surface temperature and the core body temperature, and attempts to appropriately maintain the temperature difference between the body surface temperature and the core body temperature. That is, when a peripheral blood vessel such as a fingertip vessel senses cold, the peripheral blood vessel is contracted by the action of a sympathetic nerve to reduce heat dissipation and maintain the body temperature, and the muscles produce heat and prevent the body temperature from decreasing by shaking. However, due to changes in outdoor temperature (humidity) ((extremely) low temperature outdoors, excessive air conditioning indoors), extreme diets, sarcopenia (muscle loss) due to lack of exercise, low thyroid hormone levels, etc., there may be a decrease in thermogenesis, the balance of autonomic nerves may be disturbed by stress, aging, spondylosis deformans, or the like, or the contractile function of blood vessels may be deteriorated, and thus, the balance with heat dissipation cannot be maintained, and the temperature of the whole body is lowered, resulting in hypothermia. That is, hypothermia occurs when the body's heat dissipation greatly exceeds heat production. About 60% of the heat production in the body is caused by muscles and 40% of the heat production in the body is caused by internal organs such as the liver.

On the other hand, the body can compensate to some extent for large variations in thermal load, but significant or prolonged exposure to heat beyond its ability to dissipate heat may increase core body temperature and cause heat stroke. Mild and transient core body temperature increases are tolerable, but severe increases (generally above 41 °C) may result in protein denaturation and, particularly during strenuous exercise and heavy labor at a high temperature, may result in a release of inflammatory cytokines (e.g., tumor necrosis factor α, IL-1 β). This may result in cellular dysfunction and activation of inflammatory cascades. These pathophysiological processes are similar to those of multiple organ dysfunction syndrome that follows prolonged shock (shock: multiple organ dysfunction syndrome (MODS)).

In view of the above, it is suggested that, particularly during sleep, a relatively narrow range of humidity and temperature causes comfortable sleep, but when the humidity and temperature deviate even a little from the range, they may cause sleeping difficulty, or may cause hypothermia or heat stroke in the elderly or those with underlying medical conditions, cause cerebral infarction, myocardial infarction, pulmonary infarction, or arrhythmia due to dehydration caused by excessive sweating, or cause heart failure or renal insufficiency when sweating is not possible, etc. As described above, with aging, the autonomous thermoregulation function declines and the comfortable environment range becomes narrower. In view of this, it is understood that the elderly and the patients with severe obesity, frailty, and specific complication of diseases need to actively adjust the temperature and humidity in accordance with the season, the physical condition, and each stage of sleep depending on the body temperature adjustment ability of each individual.

In addition, although the principle is basically that the head is cold and the feet are warm (cool the brain that has become warm throughout the day), in the case of physical overwork, the temperature may increase in combination with acute inflammatory changes in local muscles, and pain accompanying the temperature increase may act as noise in sleep. Indeed, bathing / massage or cold compress may also be effective. Since the local temperature control in the bed is ultimately limited to the temperature control at the contact point with the body, a quilt is also used in combination, but even so, considering turning over and body movements, in fact, when the quilt is thrown away, hypothermia is often experienced in winter or when using a cooling air conditioner, etc., and temperature control with only the conventional quilt or sleepwear is difficult.

Here, various kinds of garments capable of adjusting the (sensible) temperature and moisture of the body of the user have been proposed. For example, Patent Literature 1 discloses a body-sensible temperature adjustment garment equipped with an air fan that blows air which is dehumidified and cooled by a dehumidifying and cooling filter. Air is blown from the air fan to the inside of the garment worn by the user. Then, the air blown from the air fan spreads inside the garment around the air fan.

### Citation List

### Patent Literature

Patent Literature 1: JP 2019-199674 A

### Summary of Invention

### Technical Problem

Here, it is also assumed that the body cooling garment of Patent Literature 1 is worn during sleep, for example, in a season, etc. when the temperature is high, and the body is cooled in order to obtain comfortable sleep. However, the appropriate temperature differs for each body part (for example, the head, the neck, the arms, the legs, the abdomen, etc.). For example, there are some parts that need to be cooled considerably strongly, such as the brain, testicles, and acute inflammatory sites, and other parts that need not be cooled excessively, such as chronic inflammatory sites, lower limbs, and internal organs including ovaries, liver, and kidneys. Therefore, for comfortable sleep, it is desirable to blow air at an appropriate temperature to each body part of the user in accordance with the environment such as the season and the weather, the mental and physical conditions of the individual, and the like. However, in the technique of Patent Literature 1, it is difficult to individually blow air to each part of the body. In view of the above circumstances, an object of the present invention is to provide a garment system capable of individually and appropriately blowing air to each section corresponding to a body part of a user.

### Solution to Problem

To solve the above-described problem, a garment system of the present invention includes: a garment that is worn by a user; a blower unit that blows air toward the garment; and a control device that controls the blower unit, wherein the garment is divided into a plurality of sections respectively corresponding to a plurality of parts of a body of the user, and includes an inner layer positioned on a side of the user and an outer layer positioned on a side opposite to the user when viewed from the inner layer, the blower unit blows air between the inner layer and the outer layer of each of the plurality of sections, and the control device controls air blowing performed by the blower unit for each of the sections.

### Advantageous Effects of Invention

In the garment system according to a preferred aspect of the present invention, air blowing is controlled for each of a plurality of sections (i.e., for each part) of the garment corresponding to each body part. For example, the presence or absence of air blowing, the air blowing timing, the air volume, the temperature, the humidity, and the like can be made different for each section. That is, it is possible to appropriately blow air individually for each body part. Therefore, comfortable sleep can be provided to the user who sleeps while wearing the garment system. Consequently, without depending on chemicals or drugs and by a physical or environmental approach with relatively little individual differences, it is possible to achieve individualized sleep optimization, to radically improve the sleep quality of individuals, and to improve the productivity of activities other than sleep while preventing various diseases including infections. In addition, by realizing comfortable sleep, the cognitive function can be maintained and the healthy life span can be extended.

### Brief Description of Drawings

Fig. 1 is a configuration diagram of a garment system according to a first embodiment.
Fig. 2 is a schematic diagram of a garment according to the first embodiment.
Fig. 3 is a block diagram illustrating functions of the garment system according to the first embodiment.
Fig. 4 is an explanatory diagram of a learned model stored in a storage device according to a second embodiment.
Fig. 5 is a schematic diagram of a garment according to a modification.
Fig. 6 is a configuration diagram of a blower unit according to the modification.

### Description of Embodiments

### <First embodiment>

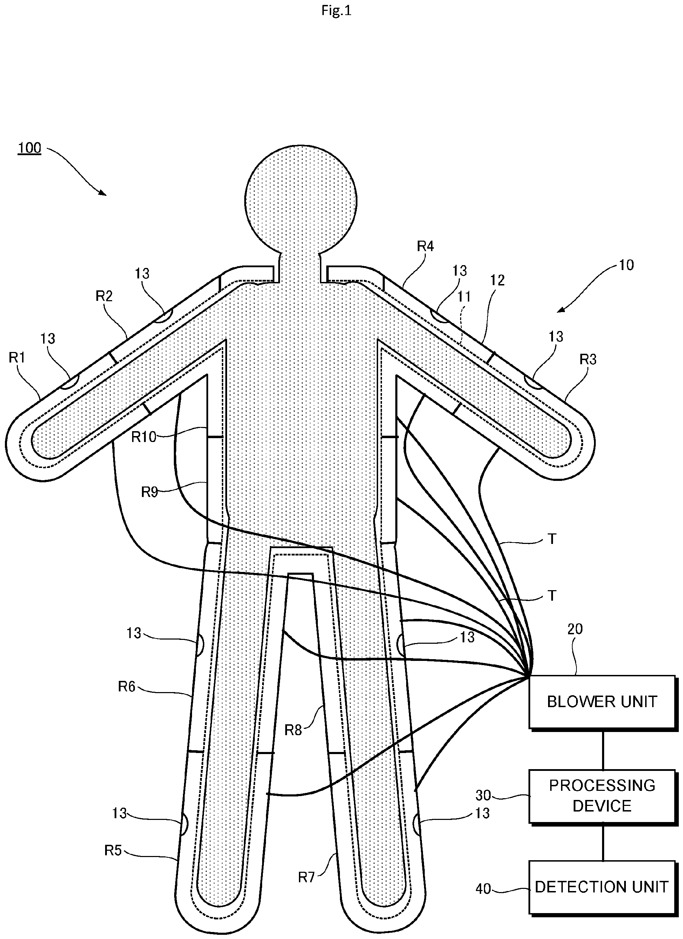

Fig. 1 is a configuration diagram illustrating a garment system 100 according to a first embodiment. The garment system 100 is related to a garment worn during sleep in order to make the user's sleep comfortable. As illustrated in Fig. 1, the garment system 100 of the first embodiment includes a garment 10, a blower unit 20, a processing device 30, and a detection unit 40.

### <Garment 10>

The garment 10 of the first embodiment is a sleeping bag-shaped garment 10 having a human shape corresponding to the shape of the body of the user. As illustrated in Fig. 1, the parts other than the head and the neck of the user are covered with the garment 10. Specifically, the garment 10 includes an inner layer 11 and an outer layer 12. The inner layer 11 is positioned on the user side in a worn state and is a region with which the user directly comes into contact. The outer layer 12 is positioned on a side opposite to the user when viewed from the inner layer 11. When viewed from the user wearing the garment 10, the outer layer 12 is positioned outside the inner layer 11. In other words, the inner layer 11 is positioned between the user and the outer layer 12.

The inner layer 11 and the outer layer 12 are sheet-like members, and are formed in a bag shape in which a portion corresponding to the neck is opened. The outer layer 12 and the inner layer 11 are connected to each other in a manner that the inner layer 11 is positioned inside the outer layer 12. For example, the inner layer 11 and the outer layer 12 are connected (for example, sewn up) at the position of the opening of the neck. Here, the inner surface of the outer layer 12 (the surface on the side facing the inner layer 11) and the outer surface of the inner layer 11 (the surface on the side opposite to the user) are not connected to each other except at the portion corresponding to the opening of the neck. That is, a space is formed between the outer surface of the inner layer 11 and the inner surface of the outer layer 12. In addition, it is assumed that air exchange between the left and right sides is difficult in the front with an opening in the center of the front so that one user can put on the garment.

The inner layer 11 and the outer layer 12 are formed of arbitrary materials (for example, fiber). It is preferable that the inner layer 11 is formed of a material having air permeability. The garment 10 is subjected to a countermeasure against parasites such as mites, lice and fleas, and a countermeasure against static electricity. Further, since the inner layer 11 is prone to stains and odors, it is necessary to facilitate replacement and washing. Thus, it is preferable that the inner layer 11 is configured to be detachable from the outer layer 12.

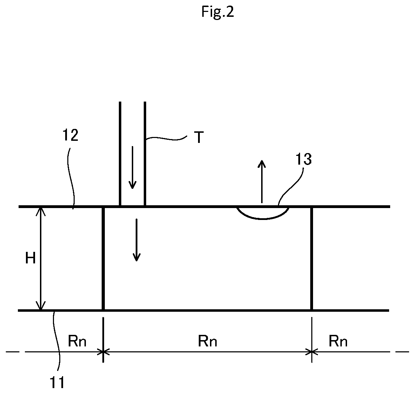

Fig. 2 is a schematic diagram that schematically illustrates the inner layer 11 and the outer layer 12. As illustrated in Figs. 1 and 2, the garment 10 is divided into a plurality of sections Rn (n = 1 to N) respectively corresponding to a plurality of parts of the body of the user. For example, the plurality of sections Rn of the garment 10 include a section R1 corresponding to the right forearm, a section R2 corresponding to the right upper arm, a section R3 corresponding to the left forearm, a section R4 corresponding to the left upper arm, a section R5 corresponding to the right lower leg, a section R6 corresponding to the right thigh, a section R7 corresponding to the left lower leg, a section R8 corresponding to the left thigh, a section R9 corresponding to the belly, and a section R10 corresponding to the thorax, etc.

Here, the number of the plurality of sections Rn of the garment 10 is arbitrary. For example, the body may be divided into 24 to 32 sections Rn on each of the front surface and the back surface and one or more sections Rn corresponding to the side surface. Similarly, the position, size, or the like of each of the sections Rn is arbitrary.

A configuration for defining each of the sections Rn of the garment 10 is arbitrary. For example, each of the plurality of sections Rn may be defined by a member (for example, a cloth-like member) that separates each of the sections Rn. In addition, the outer surface of the inner layer 11 and the inner surface of the outer layer 12 may be connected (for example, sewn up) along the peripheral edges of the sections Rn so as to define each of the sections Rn. As understood from the above description, in each of the plurality of sections Rn of the garment 10, a space H defined by the inner layer 11 and the outer layer 12 can be conceived.

### <Blower unit 20>

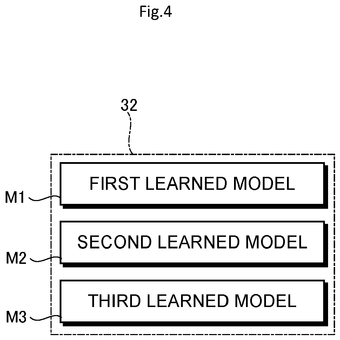

Fig. 4 is a block diagram illustrating the function of the garment system 100. The blower unit 20 is a device that blows air to the garment 10. Specifically, the blower unit 20 individually blows air to each of the plurality of sections Rn of the garment 10. Air is blown to each of the plurality of sections Rn of the garment 10 between the inner layer 11 and the outer layer 12. That is, in each of the sections Rn, air is blown from the blower unit 20 into the space H defined by the inner layer 11 and the outer layer 12. The blower unit 20 according to the first embodiment blows air whose temperature and humidity are adjusted. It is preferable that the inner layer 11 is formed of a material having high air permeability so that the air blown from the blower unit 20 is blown to the user through the inner layer 11.

As illustrated in Fig. 4, the blower unit 20 includes a blower 21, a temperature adjustment mechanism 22, and a humidity adjustment mechanism 23. The blower 21 may be, for example, various types of blower devices such as a fan, a circulator, or a compressor.

The temperature adjustment mechanism 22 is a device that adjusts (cools or heats) the temperature of the air blown by the blower unit 20. For example, the temperature adjustment mechanism 22 includes a cooling device (for example, a Peltier element) capable of cooling the air blown by the blower unit 20 and a heating device (for example, a heater) capable of heating the air blown by the blower unit 20. Here, the specific configuration of the temperature adjustment mechanism 22 is arbitrary as long as the temperature of the air blown can be adjusted. The temperature adjustment mechanism 22 can adjust the temperature of the air blown by the blower unit 20 to each of the sections Rn (parts).

The humidity adjustment mechanism 23 is a device that adjusts the humidity of (dehumidifies or humidifies) the air blown by the blower unit 20. For example, the humidity adjustment mechanism 23 may include a dehumidifier capable of dehumidifying the air blown by the blower unit 20 and a humidifier capable of humidifying the air blown by the blower unit 20. Here, the specific configuration of the humidity adjustment mechanism 23 is arbitrary as long as the humidity of the air blown can be adjusted. The humidity adjustment mechanism 23 can adjust the humidity of the air blown by the blower unit 20 to each of the sections Rn (parts).

The air blown by the blower 21 is blown to each of the sections Rn whose temperature and moisture have been adjusted by the temperature adjustment mechanism 22 and the humidity adjustment mechanism 23. The number of the blower units 20 included in the garment system 100 is arbitrary. For example, the blower unit 20 may be provided in each of the sections Rn of the garment 10.

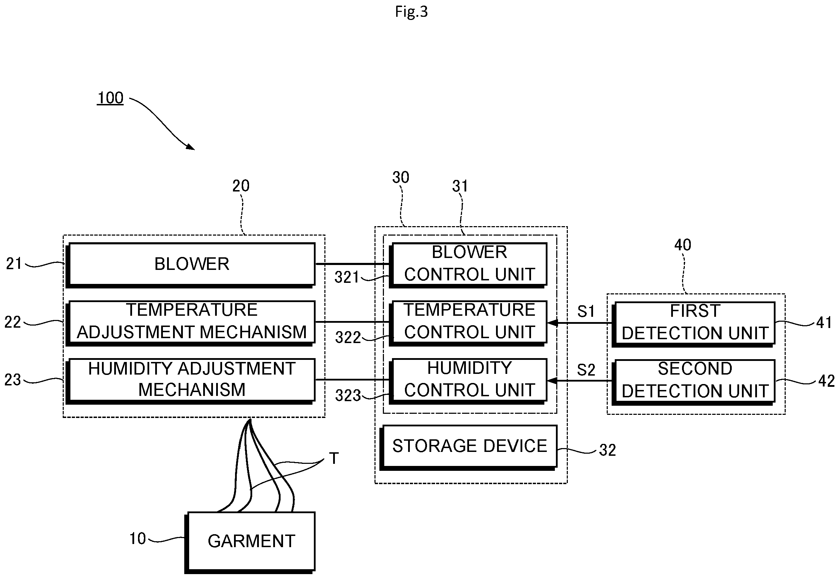

As illustrated in Figs. 1 to 3, the blower unit 20 blows air to each of the sections Rn via a tube T. One tube T is connected to each of the sections Rn of the garment 10. That is, the garment system 100 according to the first embodiment includes a plurality of tubes T respectively corresponding to a plurality of sections Rn (a plurality of parts of the body). Each of the tubes T is connected to the outer layer 12 of the section Rn corresponding to the tube T.

### <Detection unit 40>

The detection unit 40 is a mechanism that detects various kinds of information related to the living body of the user. Specifically, the detection unit 40 includes a first detection unit 41 and a second detection unit 42. The first detection unit 41 is a detection device that detects the body temperature of the user. Specifically, the first detection unit 41 detects the body temperature of each of the plurality of parts of the user. For example, an infrared sensor may be used as the first detection unit 41. Here, a thermometer that can detect a body temperature by being attached to each part of the user may be used as the first detection unit 41.

The second detection unit 42 is a detection device that detects the moisture of the body surface of the user. Specifically, the second detection unit 42 detects the moisture of the body surface of each of the plurality of parts of the user. For example, a hygrometer that can be attached to each part of the user may be used as the second detection unit 42. In the following description, a body temperature detected by the first detection unit 41 is referred to as a detected temperature S1, and a moisture detected by the second detection unit 42 is referred to as a detected moisture S2.

### <Processing device 30>

The processing device 30 is a device for controlling the blower unit 20. For example, the processing device 30 may be realized by a computer system including a control device 31 and a storage device 32. For example, an information terminal such as a personal computer or a tablet may be used as the processing device 30.

The control device 31 is, for example, configured of a single or a plurality of processing circuits such as CPUs (Central Processing Units), and integrally controls each element of the processing device 30. The storage device 32 is, for example, a single or a plurality of memories configured of a known recording medium such as a magnetic recording medium or a semiconductor recording medium, and stores a program executed by the control device 31 and various data used by the control device 31.

The control device 31 realizes various functions for controlling the blower unit 20. Specifically, the control device 31 can control the air blowing performed by the blower unit 20 to each of the sections Rn of the garment 10. As illustrated in Fig. 4, the control device 31 of the first embodiment functions as a blower control unit 321, a temperature control unit 322, and a humidity control unit 323.

The blower control unit 321 controls the blower 21 of the blower unit 20. Specifically, the blower control unit 321 controls the air blowing performed by the blower unit 20 to each of the sections Rn (that is, body part). For example, air blowing performed by the blower unit 20 is controlled in accordance with various parameters related to air blowing (hereinafter referred to as "air blowing parameters") set for each of the sections Rn. The air blowing parameters are, for example, the presence or absence of air blowing performed by the blower 21, the air volume, the timing at which the blower 21 blows air, the length of time during which the blower 21 blows air, and the like. The air blowing parameters are arbitrary and may be set in advance by the user.

The temperature control unit 322 controls the temperature adjustment mechanism 22 of the blower unit 20. For example, the temperature control unit 322 controls the temperature adjustment mechanism 22 so that the blower 21 blows air at a temperature at which each part of the user is maintained at a desired body temperature (hereinafter referred to as "target body temperature"). The target body temperature may be different for each part (section Rn). For example, the target body temperature may be set to be lower for a part close to the head (section Rn) than for a part close to the feet (section Rn).

Specifically, the temperature control unit 322 controls the temperature adjustment mechanism 22 in accordance with the detected temperature S1 detected by the first detection unit 41. When the detected temperature S1 is lower than the target temperature, the temperature adjustment mechanism 22 is controlled so as to heat the air blown by the blower 21. On the other hand, when the detected temperature S1 exceeds the target temperature, the temperature adjustment mechanism 22 is controlled so as to cool the air blown by the blower 21. The target temperature is arbitrary, and may be set by a manufacturer at the time of manufacturing the garment system 100 or may be arbitrarily set by the user.

As understood from the above description, for each of the plurality of the sections Rn, the temperature control unit 322 controls the temperature of the air blown by the blower unit 20 in accordance with the detected body temperature which is detected by the first detection unit 41 for the part corresponding to the section Rn. Therefore, air can be blown to each part at an appropriate temperature.

The humidity control unit 323 controls the humidity adjustment mechanism 23 of the blower unit 20. Specifically, the humidity control unit 323 controls the temperature of the air blown by the blower unit 20 for each of the sections Rn of the garment 10. For example, the humidity control unit 323 controls the humidity adjustment mechanism 23 so that the blower 21 blows air at a humidity at which the body surface of each part of the user is maintained at a desired moisture (hereinafter referred to as "target moisture"). The target moisture is, for example, 40% to 50% inclusive. The target moisture may be different for each part (section Rn).

Specifically, the humidity control unit 323 controls the humidity adjustment mechanism 23 in accordance with the detected moisture S2 detected by the second detection unit 42. When the detected moisture S2 is lower than the target moisture, the humidity adjustment mechanism 23 is controlled so as to humidify the air blown by the blower 21. On the other hand, when the detected moisture S2 exceeds the target moisture, the humidity adjustment mechanism 23 is controlled so as to dehumidify the air blown by the blower 21. The target moisture may be set by the manufacturer at the time of manufacturing the garment system 100 or may be arbitrarily set by the user.

As illustrated in Figs. 1 and 2, a vent hole 13 is formed in the outer layer 12 of each of the sections Rn. The air blown to each of the sections Rn is finally discharged to the outside of the garment 10 through the vent hole 13. It is not necessary to provide the vent hole 13.

As can be understood from the above description, for each of the plurality of the sections Rn, the humidity control unit 323 controls the humidity of the air blown by the blower unit 20 in accordance with the moisture detected by the second detection unit 42 for the part corresponding to the section Rn. Therefore, air can be blown to each part at an appropriate humidity.

The control by the temperature control unit 322 and the humidity control unit 323 is executed at predetermined intervals (for example, every 2 to 5 minutes inclusive). Therefore, the detection of the body temperature by the first detection unit 41 and the detection of the humidity by the second detection unit 42 are also executed at the intervals.

As understood from the above description, in the garment system 100 of the first embodiment, since the air blowing is controlled for each of the plurality of the sections Rn of the garment 10 corresponding to each part of the body (that is, for each part), it is possible to individually blow air appropriately for each part of the body. Therefore, comfortable sleep can be provided to the user of the garment system 100.

The temperature of the air blown by the blower unit 20 is controlled in accordance with the detected body temperature which is detected by the first detection unit 41, and the humidity of the air blown by the blower unit 20 is controlled in accordance with the detected moisture S2 which is detected by the second detection unit 42. Therefore, it is possible to blow air to each of the sections Rn in consideration of the body temperature and the body surface moisture of the user. Particularly, in the first embodiment, since the temperature and the humidity of the air blown by the blower unit 20 are controlled for each of the sections Rn, it is possible to blow air at an appropriate temperature and humidity to each body part of the user.

### <Second embodiment>

A second embodiment of the present invention will be described. Elements having the same actions or functions as those of the first embodiment in the embodiments illustrated below are denoted by the same reference signs as those used in the description of the first embodiment and detailed description of the elements will be omitted as appropriate.

The second embodiment is different from the first embodiment in the method of controlling the blower unit 20. Other configurations of the blower unit 20 are the same as those of the first embodiment.

The processing device 30 of the second embodiment controls the blower unit 20 using a learned model. The learned model is a statistical estimation model generated by machine learning. For example, various statistical estimation models such as decision tree or neural network may be preferably used as the learned model. The learned model is realized by a combination of a program (for example, a program module constituting artificial intelligence software) that causes the control device 31 to execute an operation of generating output data from input data and a plurality of coefficients applied to the operation. The plurality of coefficients are set by machine learning (in particular, deep learning) using a large amount of teacher data and are held in the storage device 32.

As illustrated in Fig. 4, in the second embodiment, a first learned model M1, a second learned model M2, and a third learned model M3 are stored in the storage device 32 and are used to control the blower unit 20.

The first learned model M1 is used to control the blower 21 of the blower unit 20. Specifically, the first learned model M1 is a model obtained by learning a relation between information on users during sleep (hereinafter referred to as "user information") and air blowing parameters. The user information is, for example, information on the user himself / herself (for example, physical condition, body temperature, blood pressure, and the like) and information on an environment in which the user is placed (for example, season, weather, temperature, humidity, and the like). Here, the user information is not limited to the above examples. The first learned model M1 receives the user information as an input data and outputs the air blowing parameters. The first learned model M1 is capable of outputting air blowing parameters for each of the sections Rn of the garment 10.

The second learned model M2 is used to control the temperature adjustment mechanism 22 of the blower unit 20. Specifically, the second learned model M2 is a model that has learned the relation between the user information and the target temperature. The second learned model M2 receives the user information as an input data and outputs the target temperature. The second learned model M2 is capable of outputting the target temperature for each of the sections Rn of the garment 10.

The third learned model M3 is used to control the humidity adjustment mechanism 23 of the blower unit 20. Specifically, the third learned model M3 is a model that has learned the relation between the user information and the target moisture. The third learned model M3 receives the user information as an input data and outputs the target moisture. The third learned model M3 is capable of outputting the target moisture for each of the sections Rn of the garment 10.

Similarly to the first embodiment, the control device 31 of the processing device 30 functions as the blower control unit 321, the temperature control unit 322, and the humidity control unit 323.

The blower control unit 321 controls the blower 21 as in the first embodiment. The blower control unit 321 of the second embodiment outputs the air blowing parameters by inputting the user information to the first learned model M1. Then, the blower control unit 321 controls the blower 21 according to the air blowing parameters output by the first learned model M1. That is, the blower control unit 321 controls the air blowing performed by the blower 21 in accordance with the result of inputting the user information to the first learned model M1. The user information is set in advance by the user.

Similarly to the first embodiment, the temperature control unit 322 controls the temperature adjustment mechanism 22. The temperature control unit 322 of the second embodiment outputs the target temperature by inputting the user information to the second learned model M2. Similarly to the first embodiment, the temperature control unit 322 controls the temperature adjustment mechanism 22 according to the detected temperature S1 and the target temperature.

As understood from the above description, the temperature control unit 322 according to the second embodiment controls the temperature of the air blown by the blower unit 20 in accordance with the detected temperature S1 and the result of inputting the user information to the learned model (the second learned model M2) that has learned the relation between the user information and the temperature (target temperature) of the air blown by the blower unit 20.

Similarly to the first embodiment, the humidity control unit 323 controls the humidity adjustment mechanism 23. The humidity control unit 323 according to the second embodiment outputs the target moisture by inputting the user information to the third learned model M3. Similarly to the first embodiment, the humidity control unit 323 controls the humidity adjustment mechanism 23 according to the detected moisture S2 and the target moisture.

As understood from the above description, the humidity control unit 323 controls the humidity of the air blown by the blower unit 20 in accordance with the detected moisture S2 and the result of inputting the user information to the learned model (the third learned model M3) that has learned the relation between the user information and the humidity (target moisture) of the air blown by the blower unit 20.

In the second embodiment, the same effect as that of the first embodiment is also realized. Particularly, in the second embodiment, since the blower unit 20 is controlled by the first learned model M1 that has learned the relation between the user information and the air blowing parameters, the second learned model M2 that has learned the relation between the user information and the target temperature, and the third learned model M3 that has learned the relation between the user information and the target moisture, it is possible to perform optimum air blowing in consideration of the user information.

### <Modification>

The embodiments illustrated above may be modified in various ways. Specific modification aspects will be illustrated below. It is also possible to appropriately combine two or more aspects arbitrarily selected from the following examples.
(1) In each of the above-described embodiments, the illustrated garment 10 has a sleeping bag shape, but the shape of the garment 10 is arbitrary. For example, a garment 10 shaped like a regular garment, such as a shirt or trousers, may be used in the garment system 100. As can be seen from the above description, the shape of the garment 10 is arbitrary as long as it can be worn on the body of the user. The garment 10 in the form of a sleeping bag rather than a human body may be employed. Regardless of the shape of the garment 10, the inner layer 11 and the outer layer 12 may be connected to each other such that there is a space through which the air from the blower unit 20 is introduced. In the case of the garment 10 other than the sleeping bag type, for example, the peripheral edge of the inner layer 11 and the peripheral edge of the outer layer 12 are connected to each other.
   Here, according to the garment 10 of the sleeping bag type, since parts other than the head and the neck are not exposed, there is an advantage that the garment 10 can cope with various sleeping postures and does not fall off when there is a body movement or turning over.
(2) In each of the above-described embodiments, the garment 10 may include layers different from the inner layer 11 and the outer layer 12. For example, a layer may be provided further inside the inner layer 11, or a layer may be provided further outside the outer layer 12.
(3) In each of the above-described embodiments, it is not necessary to completely separate two of the sections Rn adjacent to each other. For example, between two of the sections Rn adjacent to each other, there may be a gap as long as the air blown to one of the sections Rn does not affect the air blown to another of the sections Rn.
(4) In each of the above-described embodiments, the target temperature may be set within a range of 27.5°C to 30°C inclusive for a part having inflammation among a plurality of body parts.
(5) In each of the above-described embodiments, for example, when air is continuously blown to all the parts, it is assumed that a strong wind, which is not a breeze, is blown near the neck. Therefore, a breeze whose temperature and humidity are controlled may be intermittently blown at intervals of 5 to 10 minutes inclusive to each part, and finally, a slightly stronger wind may be constantly blown to the neck.
(6) In each of the above-described embodiments, the number of layers (for example, 3 to 6 layers inclusive) and the material are changed in accordance with the season. When there is no feverish part or sites of pain in particular, the temperature of each of the sections Rn of the garment 10 is set to gradually increase from the back surface to the front surface of the body and from the periphery of the body toward the trunk in a spiral shape. In addition, the temperature, humidity, air volume, air blowing timing, air blowing time, and the like of air blown to each part are changed and adjusted in accordance with the season, weather, room temperature, outside air temperature, humidity, and the like by utilizing the learned model so that air can be ventilated for each temperature and humidity and the same temperature is set with an error of about 0.2°C to 0.5°C inclusive.

In addition, the target temperature can be adjusted for each of the sections Rn so that the target temperature can be set higher for chronic symptoms and lower for acute symptoms for joints such as the hip, knee, upper arm, forearm, elbow, thigh, calf, and ankle joints, etc. where pain and stiffness are likely to occur. In addition, the target temperature is set to be higher for parts that should not be cooled, such as the ovary, liver, kidney, spleen, and pancreas.

The air that has passed through each part is finally discharged to the outside of the garment 10 after adjusting the temperature and moisture of the neck.

(7) In each of the above-described embodiments, the garment system 100 may be configured in consideration of the following circumstances.

Flexion of joints due to pain or the like may lead to decreased blood flow and an increase in blood pressure.

In the case of sleeping in a lower left lateral position, when there is mild or more severe heart failure, etc., since a half or more of the body is positioned above the heart, the feeling of burden and pressure on the heart may increase, and the risk of angina pectoris or the like may increase. When the user sleeps in a curled state, depending on the degree, the lungs may become difficult to expand and breathing may be suppressed.

The back muscles and spinal column are stretched as much as possible to facilitate breathing. Overlapping of parts of the body is avoided as much as possible because the temperature and moisture may increase and control of the temperature and moisture may become difficult at the overlapping sites of the parts of the body.

Particularly, it is not rare that the body temperature becomes too cold due to air conditioning and hypothermia occurs when the user sleeps with an air conditioning at about 20°C overnight in midsummer as the countermeasures against heat stroke which may occur indoors, or when the user sweats and pulls off quilts with a heating at 24°C in midwinter, etc. Hypothermia (causes: extreme diet, stress, aging, a core body temperature (not surface temperature but temperature of vital organs such as brain and heart, normally: 38.5°C for liver, 38.0°C for rectum, 37.0°C for sublingual) of 35°C or lower, wherein 32°C to 35°C inclusive is classified as mild, 28°C to 32°C inclusive is classified as moderate, and 20°C to 28°C inclusive is classified as severe), depending on complications, increases blood viscosity, reduces enzymatic reaction rates such as digestion and absorption, further leads to hypothermia, and causes a dangerous condition of falling into a vicious cycle that leads to cerebral infarction, myocardial infarction, pulmonary infarction, etc., and when the condition continues for a long time, it will increase the risk of death.

In order to surely obtain better sleep every day, the garment 10 to be worn during sleep is very important for maintaining the sensible body temperature within an appropriate temperature range for each body part.

Therefore, in the garment system 100, the body temperature and moisture of each body part including the inflammation site are monitored and controlled over time. For this purpose, the garment 10 is constituted of a plurality of layers (3 to 6 layers inclusive) each having functionality depending on the season, indoor temperature / humidity, obesity degree, basal metabolism, complications, and the like. A breeze adjusted in temperature and humidity for each body part (32 to 48 parts inclusive on each of the front (belly) side and the back side depending on the size) is blown to the inner layer 11 (innermost layer) over time through the spaces among the layers to maintain the optimum temperature and moisture.

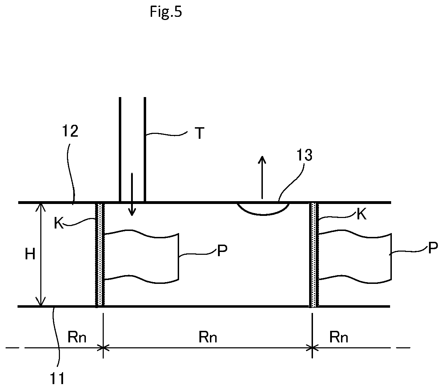

As illustrated in Fig. 5, in the inner layer 11, a large number of columnar seams (hereinafter referred to as "columnar structures") K are arranged so as to connect the outer surface of the inner layer 11 and the inner surface of the outer layer 12 in order to prevent excessive expansion, excessive depression, or excessive contraction. A cloth having a narrow width such as a streamer P is arranged on each of the columnar structures K, and the wind direction, wind force, timing and the like of the breeze are controlled and managed by a learned model so that the temperature and moisture of each part are maintained within an assumed range. Here, the length of each of the streamers P is set so that the streamer P does not reach the adjacent columnar structure K and does not become a hindrance to the change of the wind direction and the wind force by such as wrapping around the columnar structure K and wrapping or twisting one another. The functions of the layers are assumed to be heat retention, moisture absorption, heat dissipation, supplementation of weak electricity, electrode / shape retention, and the like.

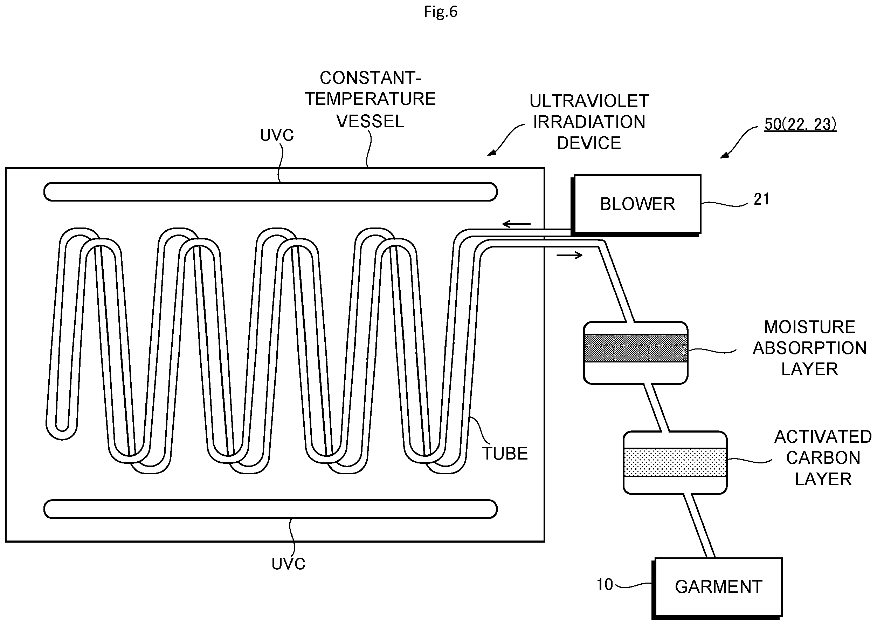

(8) It is necessary to take measures against microbial infections caused by viruses, bacteria, fungi, etc. which are not limited to the measures against COVID-19. Therefore, in the blower unit 20 according to the modification, a configuration related to measures against infections is employed. Fig. 6 is a configuration diagram of the blower unit 20 according to the modification. Specifically, the blower unit 20 includes an adjustment device 50 in which the temperature adjustment mechanism 22 and the humidity adjustment mechanism 23 are integrated. As illustrated in Fig. 6, the adjustment device 50 includes an ultraviolet irradiation device, a moisture absorption layer, and an activated carbon layer. The air blown from the blower 21 (for example, a compressor) is blown to each of the sections Rn of the garment 10 via a tube. The ultraviolet irradiation device, the moisture absorption layer, and the activated carbon layer are provided between the blower 21 and the garment 10.

The air blown from the blower 21 (for example, at a wind velocity of 0.05 to 0.1 m / sec inclusive) first passes through the ultraviolet irradiation device, and the ultraviolet irradiation device includes, for example, a constant-temperature vessel and a UVC lamp (germicidal lamp). The tube is arranged in the constant-temperature vessel set to a predetermined temperature (for example, it can be set to 18°C to 28°C inclusive in increments of 1°C), and the tube is irradiated with the UVC lamp. For example, the irradiation by the UVC lamp is performed for 10 seconds or more during which sufficient killing effect on microorganisms such as viruses and bacteria is exhibited. In addition, in the ultraviolet irradiation device, the moisture can be set to a predetermined moisture (for example, can be set in increments of 2% in a range of 40% to 52% inclusive).

The air that has passed through the ultraviolet irradiation device further passes through the moisture absorption layer (for example, silica gel) whose moisture can be adjusted, and is blown to the activated carbon layer. The activated carbon layer collects ozone generated in the ultraviolet irradiation device. Then, the air that has passed through the activated carbon layer is blown to positions corresponding to respective parts of the garment 10.

Fig. 6 illustrates a configuration in which the adjustment device 50 includes one ultraviolet irradiation device, but the number of ultraviolet irradiation devices is arbitrary and can be appropriately changed in accordance with the length of the tube. Regardless of the number of ultraviolet irradiation devices, from the viewpoint of obtaining a sufficient killing effect, a configuration that allows irradiation with the UVC lamp for 10 seconds or more as a whole is preferable.

The temperature and humidity of the adjustment device 50 are expected to change before reaching each body part. Therefore, the relation between the temperature and humidity within the adjustment device 50 and the target temperature and target moisture at each part may be learned by machine learning in a learned model for each air volume and wind velocity in consideration of the outside air temperature, humidity, weather, the body temperature of the user, and the physical condition including inflammation at each body part, etc. The target temperature and target moisture of the adjustment device 50 may be set using the learned model.

In addition, before use and during use for each of 1 (normal) to 2 (in the case of shift work, etc.) weeks, the user checks the attached comfortable sleep checklist, and meanwhile, the user's feeling of comfortable sleep and satisfaction, etc. is fed back, the target temperature and target moisture are set to the comfort mode over time, the user's limbs, body movements, turning over, etc. are monitored over time to record various parameters such as body weight, breath, snoring, body temperature change, etc. over time, and temporal, physical, or geographical changes in sleeping posture, body movement, and turning over are recorded and grasped as temporal changes on the three-dimensional coordinate axis, and the recorded information is utilized for an initial condition at the time of full-scale use, or for an aid in changing the body position. As the comfortable sleep checklist, for example, the treatment may be changed in a plurality of stages as follows.

Score of 8 or less: leave as it is
Score of 9 to 14 inclusive: require adjustment by equipment personnel
Score of 15 to 20 inclusive: require instructions such as change of prescription from an attending physician
Score of 21 or higher: require treatment by a sleep specialist or neuropsychiatrist
(10) Since the inner layer 11 is directly in contact with the user U and is greatly affected by perspiration, it is preferable that the inner layer 11 is removable and formed of a washable material.
(11) The specific configuration for the temperature adjustment mechanism 22 to adjust the temperature of the air blown by the blower unit 20 is not limited to the examples of the above-described embodiments. For example, a liquid that can be heated and cooled may be used.
(12) Perspiration observed with the lapse of time during sleep causes an increase in body surface moisture and a decrease in body surface temperature. Therefore, temperature and moisture during sleep is required to be controlled in accordance with the elapse of time in consideration of psychosomatic states such as changes in body temperature, moisture, and perspiration of each body part.

In consideration of the above circumstances, it is preferable to employ a configuration in which the temperature adjustment mechanism 22 adjusts the temperature of the air blown by the blower unit 20 over time while the user U is sleeping. In the above-described configuration, it is preferable that the garment system 100 includes any known sensor that measures whether or not the user has fallen asleep and the time that has elapsed after the user falls asleep.

Specifically, before falling asleep, the temperature adjustment mechanism 22 lowers the temperature of the air blown by the blower unit 20 so as to lower the core body temperature of the trunk as well as the head and neck so that the excited state of sympathetic nerves is suppressed and the user can fall asleep smoothly. On the other hand, when the core body temperature of the trunk continues to decrease, the body is in a hypothermic state, causing functional deterioration of internal organs and tissues, etc., and there is a possibility that fatigue recovery cannot be achieved and a feeling of fatigue is felt after getting up or a fresh and positive feeling is not felt. Therefore, the temperature adjustment mechanism 22 gradually increases the temperature of the air blown by the blower unit 20 over time from falling asleep to getting up.

In addition, the temperature adjustment mechanism 22 may control the temperature of the air blown by the blower unit 20 in accordance with the depth of sleep of the user U. For example, the temperature adjustment mechanism 22 controls the temperature of the air blown by the blower unit 20 such that the core body temperature decreases and deeper sleep (stage 3 of non-REM sleep) is increased to improve the balance of the autonomic nerve at the time of nocturnal awakening or early morning awakening due to nighttime urination, etc. or when the user U is in the stages 1 and 2 of non-REM sleep or in the REM sleep state one hour or more before the scheduled time of getting up after falling asleep, and when the user U is in the REM sleep state within one hour before the scheduled time of getting up, the temperature adjustment mechanism 22 controls the temperature of the air blown by the blower unit 20 so that the core body temperature rises.

In the above configuration, it is preferable that the garment system 100 includes a sensor that detects the sleep state of the user U or the user enters the level of comfortable sleep when getting up to give feedback and let AI learn and improve the configuration. Since it is known that most of the growth hormones secreted during the deep sleep period within 3 hours after falling asleep play important roles in maintenance of internal organs and maintenance of hair, it is necessary to ensure the deep sleep period within 3 hours after falling asleep.

The temperature adjustment mechanism 22 may be controlled in accordance with individual circumstances such as the mental and physical state of the user U before sleeping (for example, environment such as work and home, overwork, stress, and the like), the living state (for example, bathing, exercise, whether or not an addictive product is ingested, and the like), and whether or not the user U has a disease.

Similarly, it is preferable that the humidity adjustment mechanism 23 is configured to adjust the humidity of the air blown by the blower unit 20 over time during the sleep of the user U.

(11) It is also preferable that the garment system 100 according to the present invention is used to promote comfortable sleep of the user U who is in an extreme environment such as a spacecraft or a deep sea submarine.

### Reference Signs List

- 10: Garment
- 11: Inner layer
- 12: Outer layer
- 13: Vent hole
- 20: Blower unit
- 21: Blower
- 22: Temperature adjustment mechanism
- 23: Humidity adjustment mechanism
- 30: Processing device
- 31: Control device
- 32: Storage device
- 40: Detection unit
- 41: First detection unit
- 42: Second detection unit
- 50: Adjustment device
- 100: Garment system
- 321: Blower control unit
- 322: Temperature control unit
- 323: Humidity control unit
- H: Space
- K: Columnar structure
- M1: First learned model
- M2: Second learned model
- M3: Third learned model
- P: Streamer
- Rn: Section of garment
- S1: Detected temperature
- S2: Detected moisture
- T: Tube

## Claims

1. A garment system capable of controlling air blowing, the garment system comprising:
a garment that is worn by a user;
a blower unit that blows air toward the garment; and
a control device that controls the blower unit, wherein
the garment is divided into a plurality of sections respectively corresponding to a plurality of parts of a body of the user, and includes an inner layer positioned on a side of the user and an outer layer positioned on a side opposite to the user when viewed from the inner layer,
the blower unit blows air between the inner layer and the outer layer of each of the plurality of sections, and
the control device controls air blowing performed by the blower unit for each of the sections.

2. The garment system according to claim 1, the garment system further comprising:
a plurality of tubes respectively connected to the outer layer of each of the plurality of sections, wherein
the blower unit blows air between the inner layer and the outer layer of each of the sections via each of the tubes.

3. The garment system according to claim 1 or claim 2, wherein
the control device includes a temperature control unit that controls a temperature of air blown by the blower unit and a humidity control unit that controls humidity of air blown by the blower unit.

4. The garment system according to any one of claims 1 to 3, wherein
the temperature control unit controls a temperature of air blown by the blower unit for each of the sections, and
the humidity control unit controls humidity of air blown by the blower unit for each of the sections.

5. The garment system according to claim 4, the garment system further comprising:
a first detection unit that detects a body temperature of each of the plurality of parts; and
a second detection unit that detects moisture of a body surface of each of the plurality of parts, wherein
the temperature control unit controls, for each of the plurality of sections, a temperature of air blown by the blower unit in accordance with a body temperature detected by the first detection unit for a part corresponding to the section, and
the humidity control unit controls, for each of the plurality of sections, humidity of air blown by the blower unit in accordance with moisture detected by the second detection unit for a part corresponding to the section.

6. The garment system according to claim 5, wherein
the temperature control unit controls the temperature of air blown by the blower unit in accordance with a temperature detected by the first detection unit and a result obtained by inputting information related to a user to a learned model that has learned a relation between the information and the temperature of air blown by the blower unit, and
the humidity control unit controls the humidity of air blown by the blower unit in accordance with the moisture detected by the second detection unit and a result obtained by inputting the information to a learned model that has learned a relation between the information and the humidity of air blown by the blower unit.
